(19) **Europäisches Patentamt**
**European Patent Office**
**Office européen des brevets**

(11) **EP 3 113 680 B1**

(12) **EUROPEAN PATENT SPECIFICATION**

(45) Date of publication and mention
of the grant of the patent:
**31.07.2019 Bulletin 2019/31**

(21) Application number: **15724810.5**

(22) Date of filing: **23.04.2015**

(51) Int Cl.:
*A61B 5/042* (2006.01)          *A61B 5/044* (2006.01)
*G06T 17/00* (2006.01)          *A61B 90/00* (2016.01)
*G16H 40/63* (2018.01)          *A61B 5/00* (2006.01)
*A61B 5/04* (2006.01)          *A61B 34/20* (2016.01)

(86) International application number:
**PCT/US2015/027390**

(87) International publication number:
**WO 2015/164667 (29.10.2015 Gazette 2015/43)**

(54) **SYSTEM AND METHOD FOR DISPLAYING CARDIAC MECHANICAL ACTIVATION PATTERNS**

SYSTEM UND VERFAHREN ZUR ANZEIGE VON MECHANISCHEN
HERZAKTIVIERUNGSMUSTERN

SYSTÈME ET PROCÉDÉ D'AFFICHAGE DE SCHÉMAS D'ACTIVATION MÉCANIQUE DE COEUR

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.04.2014  US 201461983221 P**

(43) Date of publication of application:
**11.01.2017  Bulletin 2017/02**

(73) Proprietor: **St. Jude Medical International Holding
S.à r.l.
2449 Luxembourg (LU)**

(72) Inventors:
• **NABUTOVSKY, Yelena**
**Mt. View, California 94040 (US)**
• **RAZAVI, Hoda**
**San Jose, California 95120 (US)**

(74) Representative: **Kramer Barske Schmidtchen
Patentanwälte PartG mbB
European Patent Attorneys
Landsberger Strasse 300
80687 München (DE)**

(56) References cited:
**US-A1- 2009 254 140     US-A1- 2014 005 563**

• **DE BOECK BART WL ET AL: "Three-dimensional
mapping of mechanical activation patterns,
contractile dyssynchrony and dyscoordination
by two-dimensional strain echocardiography:
Rationale and design of a novel software
toolbox", CARDIOVASCULAR ULTRASOUND,
BIOMED CENTRAL, LONDON, GB, vol. 6, no. 1,
30 May 2008 (2008-05-30), page 22, XP021037080,
ISSN: 1476-7120**

**Description**

**BACKGROUND**

a. Field

The present disclosure relates to an electrophysiology apparatus used to measure electrical and mechanical activity occurring in a heart of a patient and to visualize the activity and/or information related to the activity in a three-dimensional (3D) model. In particular, the present disclosure relates to displaying mechanical activation patterns determined by the electrophysiology apparatus in expedient formats that facilitate data comprehension.

b. Background Art

The heart contains two specialized types of cardiac muscle cells. The majority, around ninety-nine percent, of the cardiac muscle cells are contractile cells, which are responsible for the mechanical work of pumping the heart. Autorhythmic cells comprise the second type of cardiac muscle cells, which function as part of the autonomic nervous system to initiate and conduct action potentials responsible for the contraction of the contractile cells. The cardiac muscle displays a pacemaker activity, in which membranes of cardiac muscle cells slowly depolarize between action potentials until a threshold is reached, at which time the membranes fire or produce an action potential. The action potentials, generated by the autorhythmic cardiac muscle cells, spread throughout the heart triggering rhythmic beating without any nervous stimulation.

The specialized autorhythmic cells of cardiac muscle comprising the conduction system serve two main functions. First, they generate periodic impulses that cause rhythmical contraction of the heart muscle. Second, they conduct the periodic impulses rapidly throughout the heart. When this system works properly, the atria contract about one sixth of a second ahead of the ventricles. This allows maximal contribution from the atria to the filling of the ventricles before they pump the blood through the lungs and vasculature. The system also allows all portions of the ventricles to contract almost simultaneously. This is essential for effective pressure generation in the ventricular chambers. The rates at which these autorhythmic cells generate action potentials differ due to differences in their rates of slow depolarization to threshold in order to assure the rhythmic beating of the heart.

An arrhythmia occurs when the cardiac rhythm becomes irregular, i.e., too fast (tachycardia) or too slow (bradycardia), or the frequency of the atrial and ventricular beats are different. Arrhythmias can develop from either aberrant impulse formation or aberrant impulse conduction. The former concerns changes in rhythm that are caused by changes in the pacemaker cells resulting in irregularity or by abnormal generation of action potentials by sites other than the sinoatrial node, i.e., ectopic foci. Aberrant impulse conduction is usually associated with complete or partial blockage of electrical conduction pathways within the heart. Generally, aberrant impulse formation and aberrant impulse conduction can be treated using either an implantable device (i.e., an implantable cardioverter defibrillator (ICD) or a pacemaker) or catheter ablation.

Electrophysiology studies may be used to identify these arrhythmias. In one exemplary system, a measurement system introduces a modulated electric field into the heart chamber. The blood volume and the moving heart wall surface modify the applied electric field. Electrode sites within the heart chamber passively monitor the modifications to the field and a dynamic representation of the location of the interior wall of the heart is developed for display to the physician. Electrical signals generated by the heart itself are also measured at electrode sites within the heart and these signals are low pass filtered and displayed along with the dynamic wall representation. This composite dynamic electrophysiology map (diagnostic map) may be displayed and used to diagnose the underlying arrhythmia. The diagnostic map may be superimposed on a three-dimensional (3D) model of the heart or heart chamber. The 3D model may be generated using the same measurement system that performs the electrophysiology study.

The 3D model and diagnostic map are generally produced in a step-wise process. First, the interior shape of the heart is determined. This information is derived from a sequence of geometric measurements related to the modulation of the applied electric field. Knowledge of the dynamic shape of the heart is used to generate a representation of the interior surface of the heart. Next, the intrinsic electrical activity of the heart is measured. The signals of physiologic origin are passively detected and processed such that the magnitude of the potentials on the wall surface may be displayed on the wall surface representation. The measured electrical activity is displayed on the wall surface representation in any of a variety of formats, for example, in various colors or shades of a color. Finally, a location current may be delivered to a therapy catheter within the same chamber. The potential sensed from this current may be processed to determine the relative or absolute location of the therapy catheter within the chamber. These various processes occur sequentially or simultaneously several hundred times a second to give a continuous image of heart activity and the location of the therapy device. One exemplary system for determining the position or location of a catheter in a 3D heart model is described in U.S. patent no. 7,263,397 to Hauck et al.

The measurement system can also be used to physically locate a therapy device within the heart. For example, a therapy catheter or a lead for an implantable device may be guided to an appropriate therapy location within a heart chamber to perform an ablation or pacing operation, respectively. A modulated electrical field delivered to an electrode on the therapy catheter can be used to show the location of the therapy catheter within the heart. The therapy catheter location can be displayed on the diagnostic map in real time along with the other diagnostic information. Thus, the therapy catheter location can be displayed along with the intrinsic or provoked electrical activity of the heart to show the relative position of the therapy catheter tip to the electrical activity originating within the heart itself. Consequently, the physician can guide the therapy catheter to any desired location within the heart with reference to the diagnostic map to perform the desired treatment, such as ablation. Likewise, information generated by the measurement system can be used to assist in placing the lead for an implantable device at an optimal location.

Displaying of a diagnostic map on a 3D model in a manner that expedites diagnosis and treatment can be difficult. 3D geometric models, such as 3D cardiac models of the heart, need to be shown on a two-dimensional (2D) display, such as a computer monitor, on which it is often difficult to see the entire diagnostic map at the same time. For example, in some situations the user cannot see the full picture of the diagnostic map because there are background elements of the cardiac surface geometry that are obstructed by graphical elements of the cardiac surface geometry in the foreground. Thus, the 3D geometric model typically must be rotated in order to see different areas of the model. Furthermore, if the diagnostic map has animated graphics, the user may not be able to see the entirety of the graphics simultaneously because the animation will be running on the whole model (background and foreground) when the model is rotated. As a result, certain diagnostic map features and patterns might be difficult to identify using a 3D geometric model of the heart.

Further systems for displaying the activation of a heard are known from DE BOECK BART WL ET AL: "Three dimensional mapping of mechanical activation patterns, contractile dyssynchrony and dyscoordination by two-dimensional strain echocardiography: Rationale and design of a novel software box", CARIOVASCULAR ULTRASOUND; BIOMED CENTRAL; LONDON; GB; vol. 6, no. 1, 30 May 2008 (2008-05-30), page 22, XP021037080, ISSN: 1476-7120, US 2009/254140 A1 and US 2014/005563 A1.

## BRIEF SUMMARY

The present disclosure is directed to systems and methods for generating and displaying depictions of mechanical activation data. In one embodiment, a system for displaying mechanical activation patterns of a heart comprises a data input, a processor, and an output. The data input is for receiving data from an electrophysiology apparatus. The processor is electrically connected to the data input, and is configured to calculate mechanical activation parameters from the data, generate an anatomical representation of the heart from the data, divide the anatomical representation into segments, and generate a depiction that displays magnitudes of the mechanical activation parameter relative to the segments such that performance of a plurality of segments can be simultaneously evaluated and compared. The output is for transmitting the depiction that displays magnitudes of the mechanical activation parameter relative to the segments to a display. The display is configured and arranged to facilitate clinician diagnosis based on the visualized magnitudes of the mechanical activation parameter relative to the segments, and the mechanical activation parameter comprises a time or displacement parameter.

In another embodiment, a method for displaying mechanical activation patterns of a heart comprises obtaining mechanical activation data points at a processor from a data input communicatively coupled to the processor, the data points correlated to locations on an anatomical representation; dividing the anatomical representation into segments representing different anatomical regions of the heart using the processor; assigning each mechanical activation data point to at least one segment using the processor; analyzing mechanical activation data points in each segment using the processor; displaying, on a display, a depiction representative of the magnitudes of the mechanical activation parameter relative to the segments, wherein each segment is identified by an indicator representative of the analyzed mechanical activation data points, and the mechanical activation parameter comprises a time or displacement parameter.

## BRIEF DESCRIPTION OF THE DRAWINGS

FIG. 1 is a schematic representation of a medical imaging system for generating and displaying cardiac mechanical activation data on a display screen.

FIG. 2 is a display screen of the medical imaging system of FIG. 1, showing a 3D model, electrophysiology information and a depiction of mechanical activation data.

FIG. 3 is a perspective view of the 3D model of FIG. 2 having segmentation lines superimposed on an outer surface of the model.

FIG. 4 is a schematic view of a left ventricle partitioned into segments according to the segmentation lines of FIG. 3.

FIG. 5 is a bulls-eye plot partitioned according to segments established by the segmentation lines of FIG. 4.

FIG. 6 is a data plot comprising an embodiment of the depiction of FIG. 2, showing columns of mechanical activation data that can be used to generate an icon, such as a bulls-eye plot populated with mechanical activation data.

FIGS. 7 and 8 are bulls-eye plots comprising embodiments of the depiction of FIG. 2, showing mechanical activation motion and timing mathematical information, respectively.

FIGS. 9-12 are display screens of the medical imaging system of FIG. 1, showing depictions of mechanical activation data comprising renderings having mechanical activation data superimposed over anatomical features of a heart

FIG. 13 is a schematic representation of a three-dimensional volume with data points distributed in the volume.

FIG. 14 is a representative strain versus time plot or waveform.

FIG. 15 schematically depicts the collection of data points in a beating heart.

FIG. 16 is a three-dimensional representation of a point cloud and shows lines representing the possible location of veins (for example, in a coronary sinus venous tree) determined through analysis of the points comprising the point cloud.

## DETAILED DESCRIPTION

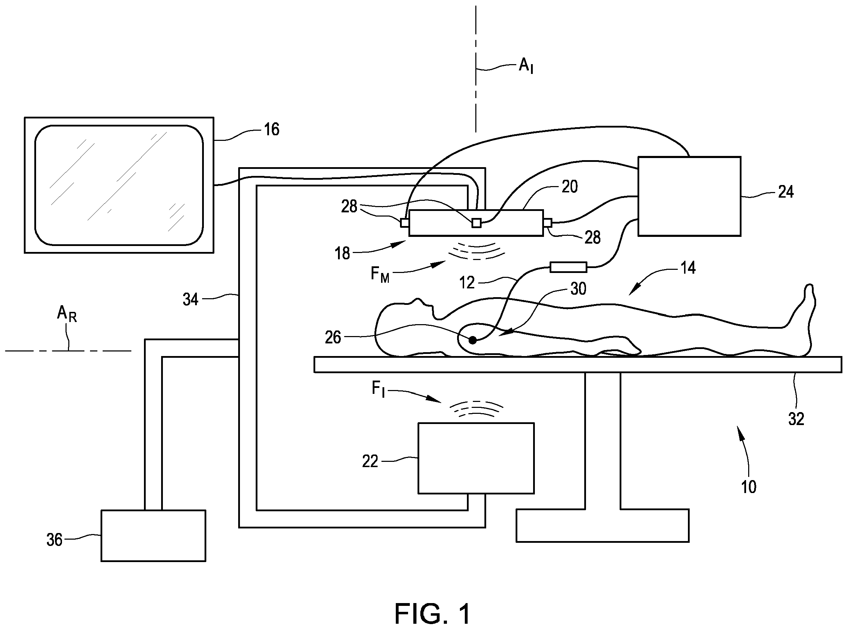

FIG. 1 is a schematic representation of medical imaging system 10 for determining the position of catheter 12 relative to a model of an organ of patient 14, as well as for generating and displaying the model and related information on display unit 16. System 10 includes moving imager 18, which includes intensifier 20 and emitter 22, and magnetic positioning system 24, which includes positioning sensor 26 (which may be called a position sensor and which may comprise, for example, one or more positioning our position coils) and field generators 28. Electrophysiology map information and cardiac mechanical activation data pertaining to the model generated by medical imaging system 10 are displayed on computer display 16 to facilitate treatment and diagnosis of patient 14. The present disclosure describes a way for system 10 to gather vast amounts of information and to synthesize the information into an easily understood format that facilitates diagnosis and treatment. For example, system 10 can be configured to collect mechanical activation data of a heart with catheter 12, and to further process that data with mathematical analysis and depict the processed data in a visual format. In one embodiment, the data are processed into an illustration, such as an icon or graph, that aggregates the data into a meaningful and accessible format. In another embodiment, the data are processed into a rendering of the heart, such as a 3D model or a 3D image, as color-coding or the like.

Moving imager 18 is a device which acquires an image of are or region of interest 30 while patient 14 lies on operation table 32. Intensifier 20 and emitter 22 are mounted on C-arm 34, which is positioned using moving mechanism 36. In one embodiment, moving imager 18 comprises a fluoroscopic or X-ray type imaging system that generates a two-dimensional (2D) image of the heart of patient 14.

Magnetic positioning system (MPS) 24 includes a plurality of magnetic field generators 28 and catheter 12, to which positioning sensor 26 is mounted at a distal end. MPS 24 determines the position of the distal portion of catheter 12 in a magnetic coordinate system generated by field generators 28, according to output of positioning sensor 26. In one embodiment, MPS 24 comprises a MediGuide gMPS magnetic positioning system, as is commercially offered by St. Jude Medical, Inc., that generates a three-dimensional (3D) model of the heart of patient 14.

C-arm 34 positions intensifier 20 above patient 14 and emitter 22 is positioned underneath operation table 32. Emitter 22 generates, and intensifier 20 receives, an imaging field $F_I$ (e.g., a radiation field) that generates a 2D image of area of interest 30 on display 16. Intensifier 20 and emitter 22 of moving imager 18 are connected by C-arm 34 so as to be disposed at opposite sides of patient 14 along imaging axis $A_I$, which extends vertically with reference to FIG. 1 in the described embodiment. Moving mechanism 36 rotates C-arm 34 about rotational axis $A_R$, which extends horizontally with reference to FIG. 1 in the described embodiment. Moving mechanism 36 or an additional moving mechanism may be used to move C-arm 34 into other orientations. For example, C-arm 34 can be rotated about an axis (not shown) extending into the plane of FIG. 1 such that imaging axis $A_I$ is rotatable in the plane of FIG. 1. As such, moving imager 18 is associated with 3D optical coordinate system having x-axis $X_I$, y-axis $Y_I$, and z-axis $Z_I$.

Magnetic positioning system (MPS) 24 is positioned to allow catheter 12 and field generators 28 to interact with system 10 through the use of appropriate wiring or wireless technology. Catheter 12 is inserted into the vasculature of patient 14 such that positioning sensor 26 is located at area of interest 30. Field generators 28 are mounted to intensifier 20 so as to be capable of generating magnetic field $F_M$ in area of interest 30 coextensive with imaging field Fi. MPS 24 is able to detect the presence of position sensor 26 within the magnetic field $F_M$. In one embodiment, position sensor 26 may include three mutually orthogonal coils (also known as position coils or positioning coils), as described in U.S. Pat. No. 6,233,476 to Strommer et al. As such, magnetic positioning system 24 is associated with a 3D magnetic coordinate system having x-axis $X_P$, y-axis $Y_P$, and z-axis $Z_P$.

The 3D optical coordinate system and the 3D magnetic coordinate system are independent of each other, that is they have different scales, origins, and orientations. Movement of C-arm 34 via moving mechanism 36 allows imaging field $F_I$ and magnetic field $F_M$ to move relative to area of interest 30 within their respective coordinate system. However, field generators 28 are located on intensifier 20 so as to register the coordinate systems associated with moving imager 18 and MPS 24. Thus, images generated within each coordinate system can be merged into single image shown on display unit 16. Moving imager 18 and MPS 24 may function together as is described in U.S. publication no. 2008/0183071 to Strommer et al.

Display unit 16 is coupled with intensifier 20. Emitter 22 transmits radiation that passes through patient 14. The radiation is detected by intensifier 20 as a representation of the anatomy of area of interest 30. An image representing area of interest 30 is generated on display unit 16, including an image of catheter 12. C-arm 34 can be moved to obtain multiple 2D images of area of interest 30, each of which can be shown as a 2D image on display unit 16.

Display unit 16 is coupled to MPS 24. Field generators 28 transmit magnetic fields that are mutually orthogonal, corresponding to axes of the 3D magnetic coordinate system. Position sensor 26 detects the magnetic fields generated by field generators 28. The detected signals are related to the position and orientation of the distal end of catheter 12 by, for example, the Biot Savart law, known in the art. Thus, the precise position and location of the distal end of catheter 12 is obtained by MPS 24 and can be shown in conjunction with the 2D images of area of interest 30 at display unit 16. Furthermore, data from position sensor 26 can be used to generate a 3D model of area of interest 30, as is described in U.S. patent no. 7,386,339 to Strommer et al.

3D models and data generated by system 10 can be used to facilitate various medical procedures. For example, it has been found that mechanical activation data, e.g., displacement of heart wall muscle, may be used in conjunction with electrical mapping data to optimize the placement of leads for cardiac resynchronization therapy (CRT) procedures. U.S. patent no. 8,195,292 to Rosenberg et al. describes exemplary methods for optimizing CRT using electrode motion tracking. However, observing, understanding, and assessing data from all the data points generated by mapping system 10 can be difficult. 3D models generated by system 10 must be rotated by a user in order to see different portions of area of interest 30. Thus, diagnosis and treatment of patient 14 can be encumbered by intermittent repositioning of the images.

The present disclosure provides systems and methods for obtaining and displaying information associated with data points collected during a medical procedure utilizing a catheter or some other medical device. In particular, heart wall motion data (e.g., displacement and timing) is displayed as a depiction (e.g., an illustration such as an icon or chart) or a 3D rendering (e.g., a 3D image or a 3D model) that shows aggregated data for various regions of the heart relative to a global representation of the heart in a single view. In one particular embodiment, the data are displayed in a bulls-eye plot after having undergone mathematical analysis.

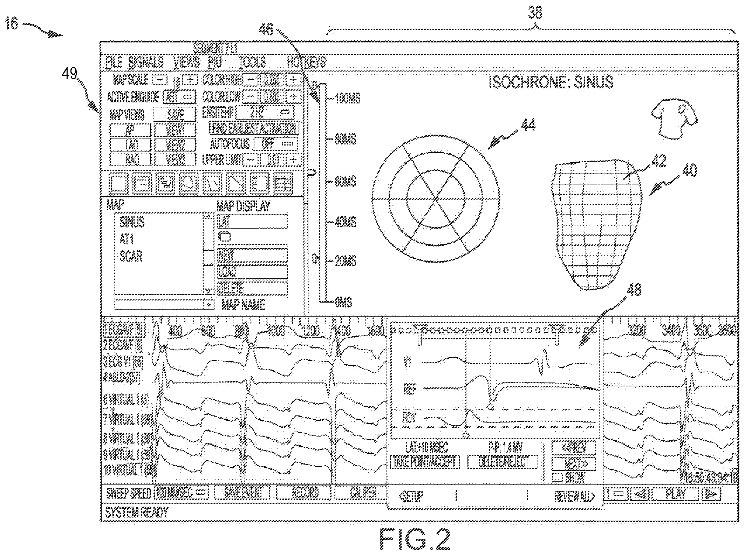

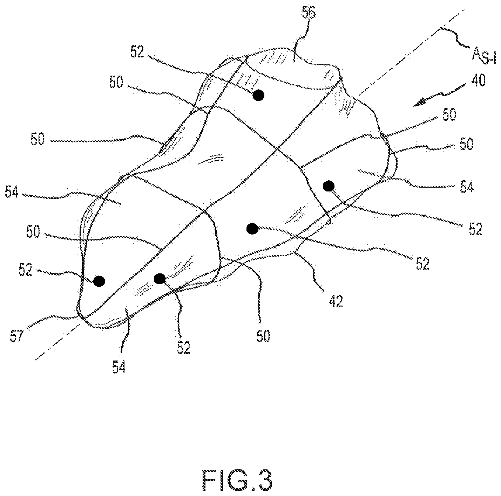

FIG. 2 is a display screen showing an exemplary image panel 38 from display unit 16 of FIG. 1. Display unit 16 is used to show data to a physician or user of system 10, and to present certain options that allow the user to tailor system configuration for a particular use. It should be noted that the contents on the display can be easily modified and the specific data presented is illustrative and not limiting. Image panel 38 shows 3D model 40 of a heart chamber, which defines surface geometry 42. Surface geometry 42 of 3D model 40 is simultaneously displayed as illustration 44 in image panel 38. However, either 3D model 40 or illustration 44 may be displayed individually. In FIG. 2, 3D model 40 is shown as a left ventricle, but may comprise any heart chamber such as an atrium or the right ventricle or any portion of a heart chamber. Additionally, system 10 may be used to map other organs of patient 14 (FIG. 1) that can be displayed on image panel 38 as a 3D model.

In the exemplary embodiment, 3D model 40 has been generated using a catheter, such as catheter 12 (FIG. 1), to create a modeled hull shape of the left ventricle. Display unit 16 also shows electrical activity of the heart of patient 14 (FIG. 1), such as the voltages associated with wave fronts of the left ventricle, that may be collected with the aid of catheter 12. 3D model 40 includes "isochrones" of electrical activity in false color (shown in grayscale in FIG. 2) associated with guide bar 46. The electrical activity can also be shown as ECG information 48. The particulars of 3D model 40 (e.g., rotation and size), illustration 44 (e.g., size, data and style) and ECG information 48 (e.g., electrograms and QRS) can be specified and/or changed at user panel 49.

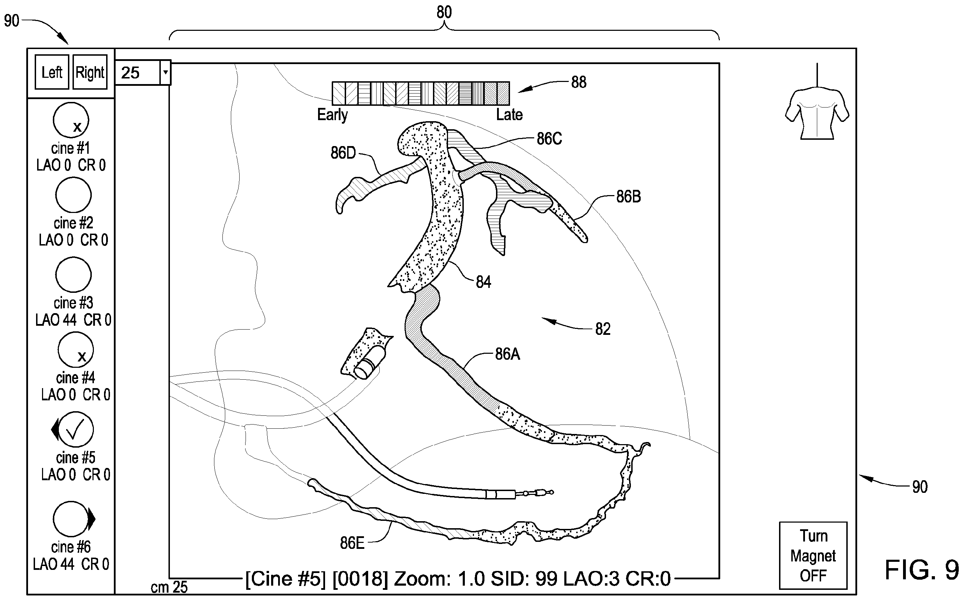

Image panel 38 shows one possible configuration of a depiction of mechanical activation data that comprises illustration 44, shown in FIG. 2 as an icon. However, illustration 44 may have various embodiments, such as a chart or plot, that provide a user of system 10 with a global view of model 40 so that data relating to foreground and background portions of surface geometry 42 may be simultaneously viewed. Illustration 44 and its various embodiments need not reproduce the exact topography of surface geometry 42, but rather provide a simplified schematic, or a graphical representation, of various regions of model 40. However, in other embodiments, depictions of mechanical activation data may comprise 3D renderings wherein the surface geometry of the 3D model, or a 3D image, is coded with indicators for illustrative purposes, as is discussed below with reference to FIGS. 9-12. Illustration 44 is divided into segments or regions that correspond to anatomical positions, such as the apex, anterior, posterior, or septal regions of a heart. Each region is then provided with a visual indication (e.g., a color, shape and/or number) corresponding to characteristics of the recorded mechanical activation activity in the region.

In one embodiment of system 10, one or more of the steps of the methods described below (e.g., with reference to FIGS. 3-8) may be performed by system 10 (e.g., embodied in software stored in memory and executed by a processor having an input connected to moving imager 18 and/or MPS 24, and an output connected to display 16) to generate 3D model 40 and illustration 44. To address the issue of identifying features and patterns of mechanical activation, an exemplary method includes the following steps: receiving collected surface data points from a medical imaging and mapping system; computing a surface hull of an anatomic structure from the surface data points; determining a mechanical activation parameter for each surface data point; assigning a plurality of segments to the surface hull; assigning each of the mechanical activation parameters to at least one segment of the surface hull; and generating a depiction showing the mechanical activation parameter for each segment. Such depictions could be useful, for example, as an aid in identifying certain global features and patterns of mechanical activations of the heart to facilitate further medical diagnostics and procedures.

Although the disclosure so far has been described with respect to a particular embodiment of medical imaging system 10, other types of imaging systems may be used to generate the 3D model and the mechanical activation data. For example, an imaging system that utilizes an electric field, rather than a magnetic field, may be used to collect the data. One such system comprises the EnSite NavX® system commercially offered by St. Jude Medical, Inc. and described in the aforementioned U.S. patent no. 7,263,397 to Hauck et al. Additionally, although the disclosure is described with respect to endocardial data processing, the concepts described herein are readily applicable to epicardial mapping, and particularly to epicardial mapping for the purposes of CRT implantations.

FIG. 3 is a perspective view of 3D model 40 of FIG. 2 having segmentation lines 50 and data points 52 disposed on surface geometry 42. Segmentation lines 50 are superimposed over surface geometry 42 to divide 3D model 40 into a plurality of segments 54. Surface geometry 42 can be shaded to show isochrones representative of electrophysiological functions of the heart, similar to what is described with reference to FIG. 2. System 10 (FIG. 1) may also generate waveforms that are aligned with respect to electrical activation from collected data, such as data points 52.

Data points 52 are scattered across surface geometry 42 and correlate to data points obtained by a user of system 10 manipulating catheter 12 in area of interest 30 (FIG. 1), such as a heart chamber. Thus, data points 52 are essentially randomly distributed over surface geometry 42 based on user input, but are collected so as to provide at least nominal coverage of various locations of interest in the heart chamber. In one embodiment, depending on the size of the heart, there are between forty and one-hundred-twenty endocardial locations and up to ten epicardial locations where recordings are made for each heart.

Each data point 52 is collected with positioning sensor 26 (FIG. 1) engaged with, or in close proximity to, a wall of the heart chamber. More precise data are collected with positioning sensor 26 lightly engaged with the heart chamber wall. However, due to movement of catheter 12 by a user, or from pumping of the heart, sensor 26 may become disengaged with the heart chamber wall for a portion of or the entire data collection period at a given map point. Beats during which the catheter is disengaged are excluded from calculations. Thus, longer recordings at each map point and a higher density of data points 52 are conducive to obtaining more accurate mechanical activation data.

Each data point 52 represents a location where catheter 12 collected one or more pieces of information pertaining to area of interest 30 of the heart chamber. Nominally, each data point 52 represents a location of tissue of the heart chamber with respect to the coordinate system of 3D model 40, as referenced by superior-inferior axis $A_{S-I}$. However, each data point 52 may be processed by system 10 to represent a mechanical activation parameter. For example, each data point 52 may be processed by system 10 to represent a maximum displacement distance for a heart chamber wall from a nominal location of 3D model 40. Additionally, each data point 52 may be processed by system 10 to represent a time it takes for a heart chamber wall to be displaced from a nominal position of 3D model 40 to the maximum displacement. Various methods for determining displacement data and timing data can be used in conjunction with the present disclosure. In one embodiment, methods known in the art are used. In another embodiment, displacement data can be determined using the methods described above with reference to FIG. 1.

As mentioned, signals from system 10 are systematically recorded at various locations of interest. In the described embodiment, data points 52 are collected at endocardial locations of interest in the left ventricle. These locations of interest are recorded relative to anatomical markers, such as left ventricular outflow tract 56 and apex 57, which are disposed relative to superior-inferior axis $A_{S-I}$ as recorded during the collection of data points 52. The anatomical markers are used to divide 3D model 40 into segments into which data points 52 are assigned for displaying in illustration 44.

3D model 40 may be divided into any number of segments that are assigned to a specific region of the heart. In the described embodiment, 3D model 40 is divided into eighteen segments 54 using two transverse segmentation lines 50 and six longitudinal segmentation lines 50 (only four of which are shown in FIG. 3). In other embodiments, 3D model 40 can be divided into twelve, sixteen, or seventeen segments, such as by dividing the apical region into fewer segments or omitting apical segments altogether. The number and layout of the segments is selected to represent a region of the heart chamber that can be unfolded or rearranged into a flat, visual illustration that shows a global view of the heart chamber. For example, in FIG. 3, an antero-septal segment at the basal end of the ventricle is defined to completely encompass left ventricular outflow tract 56. Additionally, one or more posterior segments at the basal end of the ventricle may be defined to completely encompass a left ventricular mitral annuls opposite outflow tract 56.

Each data point 52 is assigned to one or more of segments 54 for processing of illustration 44. For example, a data point 52 well within (spaced from a segmentation line 50 a distance of 1 millimeter (mm) or more) a single segment 54 is assigned to only that particular segment. However, in one embodiment, if a particular data point 52 is on a segmentation line 50 or within 1 mm of a segmentation line 50, the data point 52 is assigned to both segments 54 bordering that segmentation line 50. If a particular data point 52 is located within 1 mm of the intersection of two segmentation lines 50, the data point 52 is assigned to the four segments 54 at that intersection. The assignment of data points 52 to one or more segments 54 is used to weight statistical or mathematical data generated from data points 52 that is later displayed or represented within illustration 44.

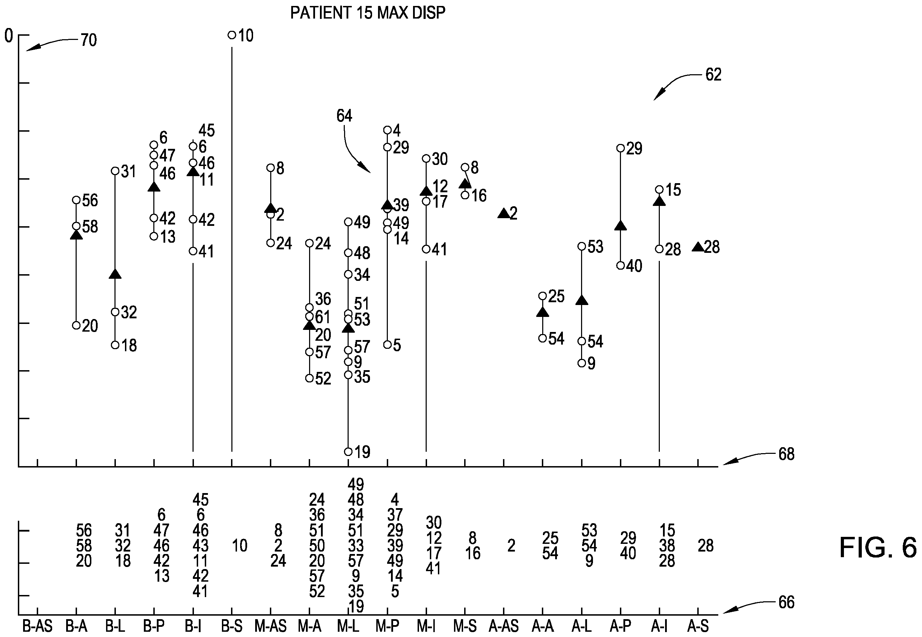

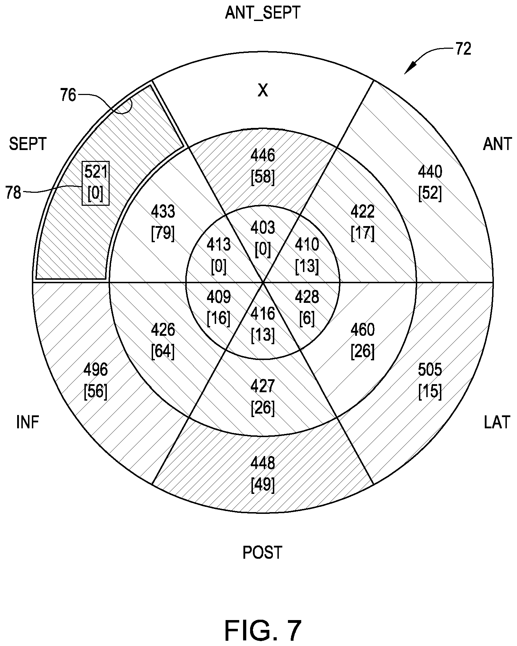

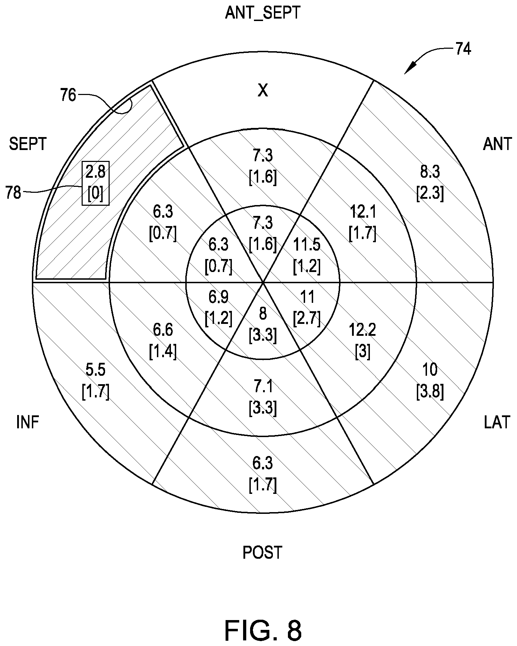

Illustration 44 (FIG. 2) of the present disclosure shows the data collected in each of the locations or segments of the heart in a visual, easy to understand format. Illustration 44 accommodates discrepancies in collected data, such as due to disengagement of the catheter with tissue, by presenting weighted averages and standard deviations of the data, with more data points producing more accurate and useful information. The weighted averages and standard deviations are presented as an indicator (colors, shapes, numbers or the like) within illustration 44, which may comprise a chart as shown in FIG. 6, or a bulls-eye plot as shown in FIGS. 7 and 8.

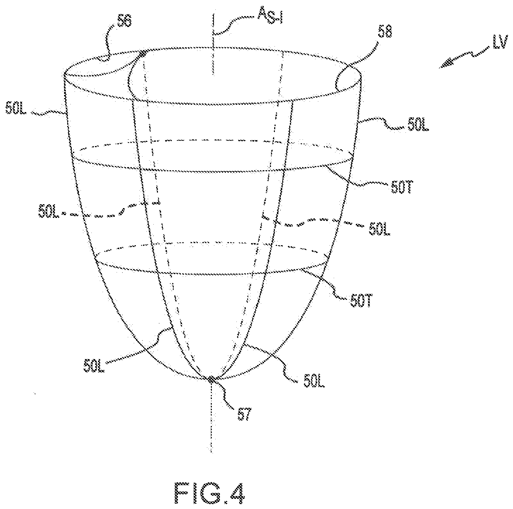

FIG. 4 is a schematic view of left ventricle LV partitioned into segments 54 according to segmentation lines 50 of FIG. 3. Left ventricle LV provides a simplified geometry of 3D model 40 useful in understanding the segmentation of the left ventricle and the generation of a resulting bulls-eye plot. Superior-inferior axis $A_{S-I}$ is drawn to extend from apex 57 of left ventricle LV to midway between all pairs of walls - anteroseptal and posterior, anterior and inferior, septal and lateral. Superior-inferior axis $A_{S-I}$ is also positioned (i.e., midway between the anterior and inferior walls) such that a plane extending through the axis bisects each of left ventricular outflow tract 56 and left ventricular mitral annulus 58.

Transverse segmentation lines 50T divide left ventricle LV into three transverse slices that extend radially from the superior-inferior axis $A_{S-I}$ and that are disposed contiguously along superior-inferior axis $A_{S-I}$. The slices are formed by a lower cutting line that extends through the region of apex 57 of 3D model 40, and an upper cutting line that extends through the mitral annulus region of 3D model 40. Thus, an apical slice is located near the bottom of 3D model 40 (with reference to the orientation of (FIG. 4); a basal slice is located near the top of 3D model 40 (with reference to the orientation of FIG. 4); and a mid-ventricular slice is located between the apical and basal slices.

Longitudinal segmentation lines 50L divide 3D model 40 into six longitudinal slices that extend longitudinally in the direction of superior-inferior axis $A_{S-I}$ and that are disposed contiguously around the circumference of 3D model 40 relative to superior-inferior axis $A_{S-I}$. Starting at the location of left ventricular outflow tract (LVOT) 56 and moving clockwise relative to FIG. 4, the longitudinal slices are as follows: antero-septal, septal, inferior, posterior, lateral, and anterior. LVOT 56 should be in the center of the antero-septal wall.

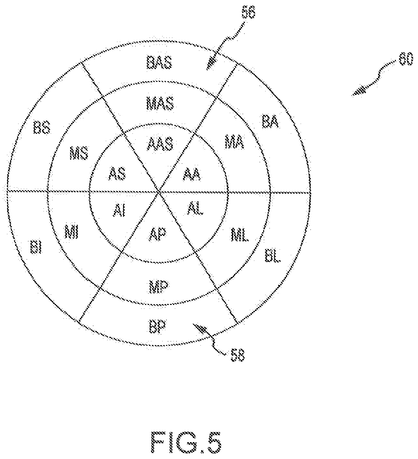

For the described embodiment, transverse segmentation lines 50T and longitudinal segmentation lines 50L divide left ventricle LV into eighteen segments: basal antero-septal (BAS), basal septal (BS), basal inferior (BI), basal posterior (BP), basal lateral (BL), basal anterior (BA), apical antero-septal (AAS), apical septal (AS), apical inferior (AI), apical posterior (AP), apical lateral (AL), apical anterior (AA), mid-ventricular antero-septal (MAS), mid-ventricular septal (MS), mid-ventricular inferior (MI), mid-ventricular posterior (MP), mid-ventricular lateral (ML), and mid-ventricular anterior (MA), as shown in FIG. 5.

FIG. 5 is bulls-eye plot 60 partitioned according to segments established by segmentation lines 50 of FIG. 4. FIG. 5 represents a view of left ventricle LV of FIG. 4 as viewed from below, looking up. Bulls-eye plot 60 is formatted similarly to standard bulls-eye plots familiar to those who are experienced in echocardiography. Bulls-eye plot 60 is drawn as a series of three concentric circles, each circle divided into six equal parts, thus creating a summary of eighteen segments of 3D model 40. Each of the eighteen segments of bulls-eye plot 60 is labeled with an anatomical reference, which is replaced with mechanical activation information to generate an embodiment of illustration 44, as shown in FIGS. 7 and 8.

FIG. 6 is a data plot 62 for data points 52 of FIG. 3. Data plot 62 comprises one embodiment of illustration 44 that can be shown in image panel 38 of display 16 in FIG. 2. Data plot 62 comprises columns 64 of mechanical activation data that can be used to generate a further embodiment of illustration 44, such as a bulls-eye plot. The bottom of data plot 62 includes labels 66, which correspond to segments 54 of model 40 identified in FIG. 5 in bulls-eye plot 60. Bottom axis 68 of data plot 62 includes hash marks aligned with one of columns 64 and one of labels 66. Longitudinal axis 70 shows values for mechanical activation data. In the depicted embodiment, the longitudinal axis indicates the maximum negative displacement (inward motion) of a heart chamber wall. However, in other embodiments, the longitudinal axis may indicate activation timing of a heart chamber wall or other parameters of interest. Furthermore, in other embodiments, data plot 62 may be configured as a box plot.

Each of columns 64 includes one or more identification numbers associated with a circle, which represents one of data points 52. The identification numbers are simply to uniquely identify each of data points 52 with respect to 3D model 40, and do not correspond to a magnitude of a mechanical activation parameter. The identification numbers are shown below bottom axis 68 to readily indicate the number of data points that went into generating each of columns 64 and to illustrate their order with respect to the parameter plotted. Within data columns 64, each identification number is shown proximate a circle located with respect to the magnitude of the mechanical activation parameter as indicated by longitudinal axis 70. Thus, the spread of the data is shown by the length of each of columns 64 so that a viewer of data plot 62 can easily see if data points 52 are consistent in each segment 54. The identification numbers are shown in the same order in columns 64 and below bottom axis 68. Because data points may lie close to segmentation lines 50, each unique identification number may be used more than once in data plot 62.

The mean of the magnitude of each circle is shown by a triangle at its appropriate position along longitudinal axis 70. In one embodiment a weighted mean is calculated, as determined by Equation [1] shown below, given values at points 1 through n: $[a_1, a_2, .... , a_n]$, and given weights at points 1 through n: $[w_1, w_2, .... ,w_n]$. Weights are determined based on how many segments 54 each data point 52 falls into. If a point is only in one segment, it has a weight of 1. If a point is in two segments, it has a weight of 0.5 in each segment. If a point falls into four segments, it has a weight of 0.25 in each segment.

$$Weighted\ Average = \frac{\sum_{i=1}^{n} w_i\, a_i}{\sum_{i=1}^{n} w_i} \qquad\qquad \textbf{Equation [1]}$$

In other embodiments, the triangle may represent a different statistical calculation for data points 52 in each of columns 64. For example, a standard deviation may be calculated. Standard deviation is useful in illustrating the variability of data points 52 within each segment 54. High variability possibly indicates less reliable data, such as that generated while positioning sensor 26 (FIG. 1) is disengaged with tissue of the heart. In one embodiment a weighted standard deviation is calculated, as determined by Equation [2] shown below.

$$Weighted\ Standard\ Deviation = \sqrt{\frac{\sum_{i=1}^{n} w_i(a_i - Weighted\ Average)^2}{\frac{n-1}{n}\sum_{i=1}^{n} w_i}} \qquad\qquad \textbf{Equation [2]}$$

Data plot 62 thus provides a global view of all of the mechanical activation data collected with reference to 3D model 40 for either displacement or timing. Thus, data plot 62 provides a first level of condensation for the vast amount of the raw data collected by system 10. The data and information presented in data plot 62 can be further condensed or distilled into a simplified and accessible format, such as an icon or graphic, so that users (e.g., a clinician) can make easy and quick judgments regarding the earliest and latest sites of activation, the activation pattern, and the reliability and consistency of the original data.

FIGS. 7 and 8 are bulls-eye plots 72 (timing) and 74 (motion or displacement), respectively, comprising embodiments of illustration 44 of FIG. 2 showing mechanical activation timing and extent of motion, respectively. For the described embodiment, each segment in the bulls-eye plots includes two numbers and is color-coded. The upper number represents a weighted mean, and the lower number represents a weighted standard deviation. The color coding is based on the value of motion extent or timing in the corresponding segment. The color coding is represented with cross-hatching in FIGS. 7 and 8.

The timing plot of FIG. 7 is color-coded, although represented in FIG. 7 as cross-hatching. Specifically, a first color (e.g., red) indicates the earliest activated segments (e.g., the following segments in FIG. 7: MA, AA, AL, AP, AI, AS, AAS, MP, and MI) and a second color (e.g., purple) indicates the latest activated segments (e.g., segment BS in FIG. 7). Other colors can be used to indicate activation timing between the earliest and latest segments such that a color scale is applied to the timing values. A segment border 76 or number border 78 can also be used to indicate timing abnormalities, or other abnormalities. For example, if a segment contains some points with outward motion and some with inward motion, an indication such as an extra border around the value indicates that some points within a segment show outward motion. For example, the basal-septal segment includes a border 78 around the upper and lower values to indicate that at least one value of outward motion was collected. If no values are available for a given segment (e.g., the BAS segment in FIG. 7), the segment will be left white, or another color that is not part of the color scale.

For the extent of motion plot of FIG. 8, all segment averages above a threshold (such as 3 mm), are colored the same color (e.g., dark purple) to indicate normal/healthy extent of motion (e.g., all of the segments except for the BAS and BS segments in FIG. 8). All segment averages that show outward motion (negative displacement) are colored another color (e.g., blue) to indicate outward motion (e.g., the BS segment in FIG. 8). The rest of the points are adjusted such that a color scale is applied to the values, and the user can quickly interpret the range of low and high displacements and their locations. Borders 76,78 can be used to highlight abnormal displacement values. If a segment contains some points with outward motion and some with inward motion, only the average of the inward motion values is displayed, but an indication such as an extra border 76 around the value, indicates that some points within a segment show outward motion. If no values are available for a given segment (e.g, the BAS segment in FIG. 8), the segment will be left white, or another color that is not part of the color scale.

Based on these plots from endocardial or epicardial motion mapping, the user will be able to quickly determine the sites of earliest and latest activation, sites of outward rather than normal inward motion, and sites of low or impaired motion. In the patient of FIGS. 7 and 8, activation traveled from the apex, to mid-inferior, posterior, and anterior sections, and then to the base of the heart. The latest site of activation is at the basal septum, which also had outward motion. The extent of motion was healthy in all segments, except the basal septal segment. A user or clinician can use this information to provide further medical assessments. In one embodiment, system 10 can automatically identify segments with irregular mechanical activation patterns and highlight the segments in display unit 16 (FIG. 1). For example, upon determining that a segment has outward (positive) motion, the segment can be highlighted, such as with border 76 around the respective segment in the bulls-eye plot.

The data summarized in bulls-eye plots 72 and 74 can be used to assist in diagnosis and other medical procedures such as left ventricular (LV) lead placement for CRT procedures. The clinician observing bulls-eye plots 72 and 74 can make a quick decision based on these illustrations as to where the LV lead should be placed. For example, the clinician would be able to identify the segment with the greatest delay (i.e., the basal septum) to mechanical activation and may choose to place the LV lead at that site.

In another example, the clinician may only have a few choices of sites due to venous anatomy, so he/she might compare motion at the available sites to see which one is later and/or which one has the greater extent of motion.

Summarizing motion data in an illustration as described above provides a tool for quick and intuitive interpretation of heart wall motion data as measured by various medical imaging systems, such as the MediGuide system. The illustration can show both extent and direction of motion, as well as the timing of the motion. The illustrations can be configured in familiar formats, such as bulls-eye plots, to display mechanical activation data. The illustrations also include an indication of the integrity of the data generated by the medical imaging system, such as by showing statistical or mathematical analysis of the data. The data can be interpreted by a user or clinician to identify sites of earliest and latest activation. The data can further be interpreted to provide information for performing other medical procedures, such as for arrhythmia ablation.

FIGS. 9-12 are display screens of medical imaging system 10 of FIG. 1, showing depictions of mechanical activation data comprising renderings having mechanical activation data superimposed over anatomical representations of a heart. FIGS. 9-12 show mechanical activation data useful in, for example, left ventricle lead implantation during cardiac resynchronization therapy (CRT) procedures.

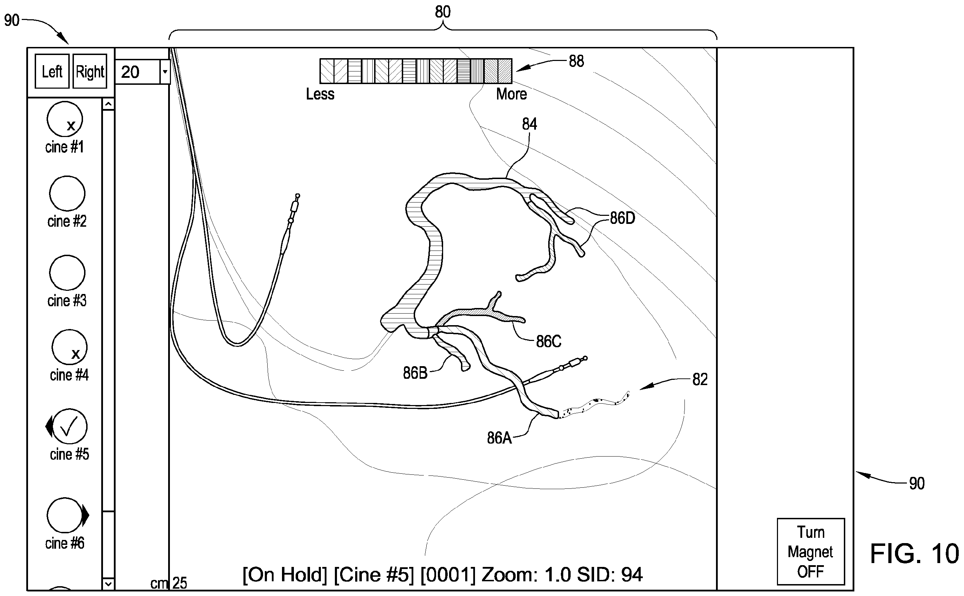

FIG. 9 is a display screen showing an exemplary image panel 80 from display unit 16 of FIG. 1. Display unit 16 is used to show data to a physician or user of system 10, and to present certain options that allow the user to tailor system configuration for a particular use. It should be noted that the contents on the display can be easily modified and the specific data presented are illustrative and not limiting. Image panel 80 shows 3D image 82 of a heart chamber, which defines anatomical representations of coronary sinus 84 and branches 86A-86E. Sinus 84 and branches 86A-86E are shown rendered with an indicator to show mechanical activation data. In particular, branches 86A-86E are rendered in different colors indicating different magnitudes of a mechanical activation parameter (see guide bar 88). In Fig. 9, that mechanical activation parameter is timing. Also, in FIG. 9, as in FIGS. 10-12, colors are represented by various crosshatching patterns. The particulars of 3D image 82 (e.g., rotation, size, and zoom), and the rendering (e.g., segments, colors, and style) can be specified and/or changed at user panel 90.

In the exemplary embodiment, 3D image 82 has been generated using an imager, such as moving imager 18 (FIG. 1), to create a view of the coronary sinus. As such, image 82 can be a fluoroscopy image used by the aforementioned MediGuide system. However, other types of systems may be used to generate a 3D image, such as computed tomography (CT), magnetic resonance imaging (MRI), multi-angled fluoroscopy images, 3D electrocardiography images, and electro-anatomical mapping techniques. In FIG. 9, 3D image 82 is shown as a coronary sinus of the left ventricle, but may comprise any heart chamber or features. Additionally, system 10 may be used to map other organs of patient 14 (FIG. 1) that can be displayed on image panel 80 as a 3D image.

In one embodiment of system 10, one or more of the steps of the methods described below (e.g., with reference to FIGS. 9-12) may be performed by system 10 (e.g., embodied in software stored in memory and executed by a processor having an input connected to moving imager 18 and/or MPS 24, and an output connected to display 16) to generate 3D image 82 and its associated mechanical activation data. To address the issue of identifying features and patterns of mechanical activation, an exemplary method includes the following steps: obtaining mechanical activation data points correlated to locations on an anatomical representation; dividing the anatomical representation into segments representing different anatomical regions of the heart; assigning each mechanical activation data point to at least one segment; analyzing mechanical activation data points in each segment; and displaying a depiction representative of the segments, wherein each segment is identified by an indicator representative of the analyzed mechanical activation data points. Such depictions could be useful, for example, as an aid in identifying certain global features and patterns of mechanical activations of the heart to facilitate further medical diagnostics and procedures.

Image panel 80 shows one possible configuration of a depiction of mechanical activation data comprising a rendering of an anatomical representation, shown as 3D image 82 in FIG. 9. However, in other embodiments, the rendering may comprise a 3D model, such as can be generated with catheter 12 of system 10 (FIG. 1). Each rendering may provide a user of system 10 with a three-dimensional picture of image 82 so that aggregated mechanical activation data relating to foreground and background portions of branches 86A-86E can be perceived. Mechanical activation activity may be obtained using methods known in the art. In another embodiment, displacement data can be determined using the MediGuide (MDG) system as previously discussed. For example, a MediGuide-enabled tool (MDG-enabled tool) is maneuvered around the coronary sinus (CS) and its branches, and the 3D location of the MDG sensor is tracked for 15 seconds in multiple locations along the length of each branch. For each patient, 30-50 different locations within the CS vascular tree have been tested. As discussed further below, this location data may be used in an alternative technique for determining and representing mechanical activation activity (e.g., displacement or timing) on a representation of a region of interest such as a heart chamber.

When epicardial mapping is conducted in the CS and its branches, as is done in a CRT implant procedure, anatomical landmarks located therein are used to create the segmentation. In particular, during a CRT implant, there is no access to inside the LV; and, as such, the position of the LV apex for segmentation purposes can be approximated by the RV apex on the septum instead. Similarly, the mitral annulus is not accessible during a CRT implant and, instead, anatomical markers in the main coronary sinus are used to delineate the base of the LV. 3D image 82 is divided into segments or regions that correspond to anatomical positions. In FIG. 9, the entirety of each branch comprises a single segment. Each branch is then provided with a visual indication (e.g., a color, shape, and/or number) corresponding to recorded mechanical activation activity in the region. For example, areas experiencing early timing may be identified with light colors (e.g., yellow, which is indicated in FIG. 9 using the fifth crosshatch pattern from the left-hand end of the guide bar 88, which is the crosshatch pattern shown on branch 86D), and areas experiencing later timing may be identified with dark colors (e.g., purple, which is indicated in FIG. 9 using the second crosshatch pattern from the right-hand end of the guide bar 88, which is the crosshatch pattern shown on a portion of branch 86B).

The color coding may be continuous or binary such that if a particular location reaches a certain threshold with respect to its motion, it is delineated using color or texture, for example. Regions of scar can be indicated on 3D image 82 as well to show which areas do not need to be mapped or considered for lead placement. This can be done by, for example, greying out portions of coronary sinus 84 or branches 86A-86E that overlay the scar region or just drawing a rectangle or another shape of a distinct color over the scar region.

In the presence of multiple images, different color-coding can be done on the different images to indicate a combination of motion parameters such as extent and timing of mechanical motion, as is discussed with reference to FIG. 10.

FIG. 10 is a display screen showing an alternative embodiment of 3D image 82. FIG. 10 includes the same reference numerals as discussed with reference to FIG. 9, with various branches 86A, 86B, 86C, and 86D shown in different colors (which, again, are depicted in this black-and-white figure using a variety of different crosshatch patterns). Thus, as previously mentioned, FIG. 10 may show mechanical activation displacement simultaneously while FIG. 9 shows mechanical activation timing. Both versions of 3D model 82 may be shown in image panel 80 simultaneously or alternatively to facilitate diagnoses. In FIG. 10, less displacement may be provided with light colors (e.g., yellow), while more displacement may be provided with dark colors (e.g., purple). In other embodiments, timing and displacement may be shown in colors exclusive to the image in which they are used. For example, FIG. 9 may be shown in primary colors, while FIG. 10 is shown in pastel colors.

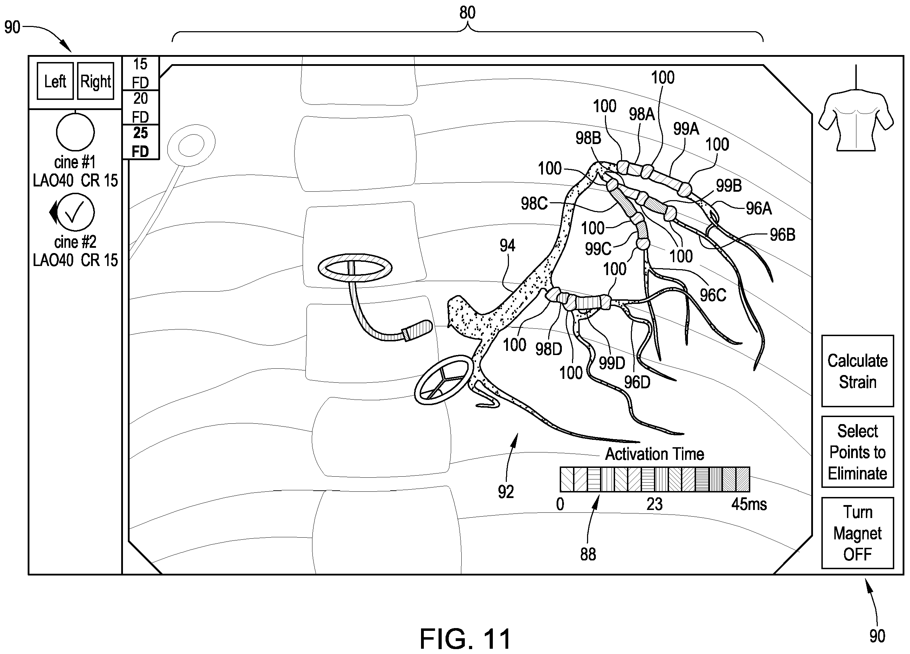

FIG. 11 is a display screen showing another embodiment of a 3D image 92. 3D image 92 is shown in image panel 80 alongside guide bar 88, and two user panels 90 straddle the image panel 80. 3D image 92 includes main CS 94 and branches 96A, 96B, 96C, and 96D. FIG. 11 is similar to the embodiment of FIG. 9, except each of branches 96A, 96B, 96C, and 96D is sub-divided into smaller segments for displaying mechanical activation data. As shown in this figure, element numbers that comprise the same capital letter (i.e., A, B, C, D) relate to, or identify features and aspects of, the same CS branch. Thus, for example, 96A, 98A, and 99A all relate to one CS branch; and 96B, 98B, and 99B all relate to a different CS branch. In particular, branch 96A includes segments 98A and 99A, branch 96B includes segments 98B and 99B, branch 96C includes segments 98C and 99C, and branch 96D includes segments 98D and 99D. Thus, the embodiment of FIG. 11 allows for higher data resolution as compared to the embodiment of FIG. 9. As such, guide bar 88 can be provided with more precise information, such as actual timing values. As depicted in Fig. 11, for example, guide bar 88 includes activation times varying from 0 to 45 ms.

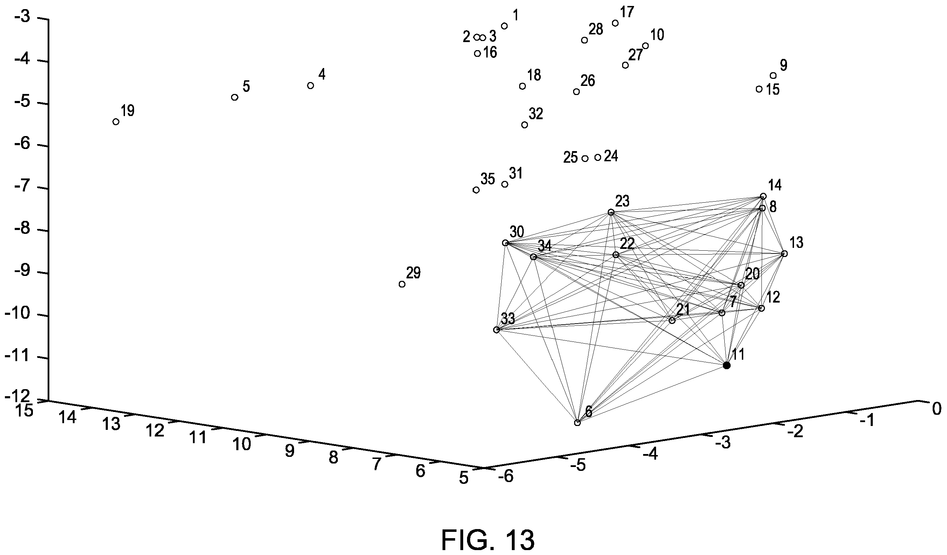

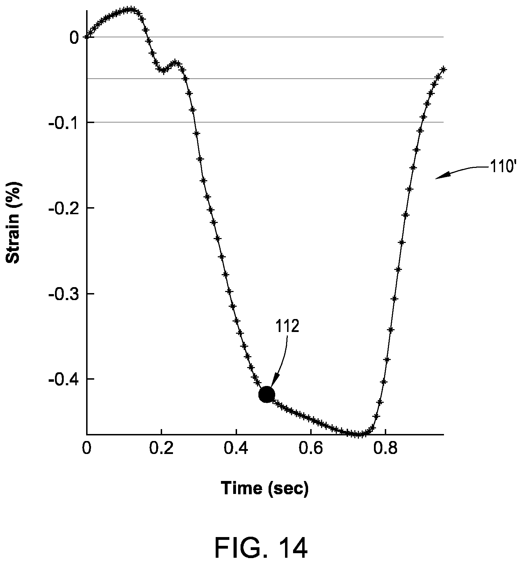

Each of branches 96A, 96B, 96C, and 96D is depicted in FIG. 11 as overlaid with different colors (as previously mentioned, colors are represented in FIGS. 9-12 by various crosshatching patterns) reflecting the differences in timing and/or extent of motion/contraction in different parts of the branch. FIG. 11 illustrates an example in which the segments between points 100 are colored according to activation time determined from strain between pairs of points 100 along the same CS branch. Thus, in this embodiment as it is depicted in FIG. 11, strain may be determined between points 100 in the same CS branch - for example, determining strain in CS branch segment 98A and branch segment 99A, since each of these CS branch segments exists between two points 100 where wall motion data is available. Another techniques for using strain to determine, for example, mechanical activation activity is discussed below in connection with FIGS. 13 and 14, where the data points used to calculate strain can reside in different CS branches (i.e., may have been captured from locations along different CS branches).

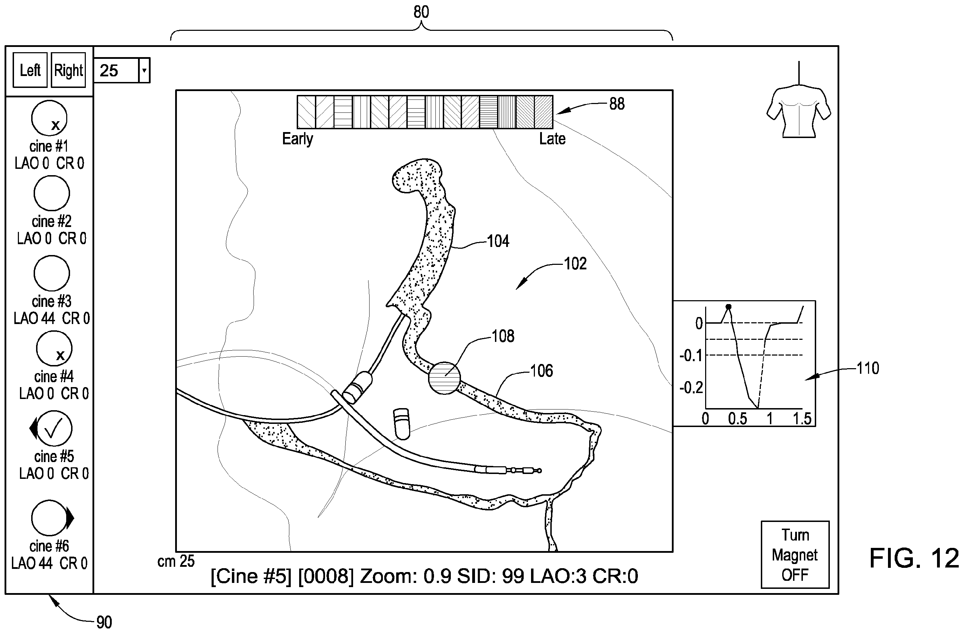

In FIG. 12, and in contrast to what is shown in FIG. 11, only the point that represents the location of the motion point may have a color representative of timing/extent of motion at that point. FIG. 12 is a display screen showing another embodiment of a 3D image. In particular, 3D image 102 is shown in image panel 80 alongside guide bar 88 and user panel 90. 3D image 102 includes main CS 104 and branch 106. Data point 108 is located on branch 106. FIG. 12 also shows waveform 110 located on the display screen. FIG. 14, which is explained further below, depicts a similar, but enlarged, waveform 110'. In the embodiment of FIG. 12, waveform 110 shows the specific motion waveform (here, "motion" is being represented by a waveform showing strain as a function of time) for data point 108. Alternatively, a stack of waveforms from different motion points can be displayed in conjunction with one or more of the above embodiments to convey contractility behavior at each segment/wall/region of chamber. For example, strain waveforms can be shown on top of or next to color-coded shapes (such as the crosshatched circle shown in FIG. 12) on images (e.g., FIGS. 9-11) to convey the contraction pattern or tendencies of the myocardium along each branch.

As mentioned, FIGS. 9-12 are useful, for example, in facilitating left ventricular lead implantation during CRT procedures. LV leads are placed at epicardial locations relative to the coronary sinus, endocardially with respect to the pericardial sac. In particular, the LV lead is placed in one or more branches of the coronary sinus. Optimally, the LV lead is placed coincident with the area having the latest activation of the left ventricle. Hemodynamic benefits are most fully utilized by placing the LV lead in the area having the latest activation (e.g., an area that, while still capable of good contraction, tends toward having a later activation time than that of other areas). Additionally, locations of scar tissue are sub-optimal for LV lead placement. The superimposition of mechanical activation data onto a 3D image, such as a fluoroscopy image such as those shown in FIGS. 9-12, allows for the identification of optimal LV lead placement locations, while simultaneously viewing sub-optimal locations.

In an embodiment, an important parameter for determining mechanical activation timing is the strain between adjacent portions of the myocardium. As described above, strain may be calculated between adjacent portions along a single branch of the coronary sinus vasculature. It may also be beneficial to determine the strain between adjacent portions of the myocardium that are not necessarily located along a single branch of the coronary sinus. FIG. 13 schematically depicts part of one technique for doing this. This figure depicts, in a three-dimensional coordinates system, thirty-five sample locations where data has been captured. These locations may include points in different branches of the coronary sinus vasculature, and each location is represented by a small circle with a reference number adjacent to it. In this example, the location represented by the number "11" is the location of particular interest. Thus, the circle representing that particular location and data is filled-in rather than being open. In this technique, and as also represented in FIG. 13, the system connects the location of interest (here, data point "11") to every adjacent location within a predetermined distance (e.g., 4-10 mm) of the location of interest to define a plurality of triangular regions of myocardium (e.g., all combinations of locations and all possible triangles). Then, by considering how, for example, the area of each of these various triangular regions changes during a cardiac cycle (and, potentially, during a number of cardiac cycles), it is possible to determine strain in the myocardium as a function of time at a particular location of interest (or a series of locations of interest). The determined strain at a particular location may then be plotted as a function of time. FIG. 14 is an example of such a curve, depicting strain as a function of time at a particular location (here, at location "11") during a cardiac cycle. By calculating how the area of these triangles changes during the cardiac cycle, it is possible to create a series of figures similar to FIG. 14, showing how the strain is changing during a cardiac cycle as an indicator of mechanical activation activity.

At time zero in FIG. 14, the particular chamber (e.g., the left ventricle) comprising the particular portion of myocardium that encompasses location "11" may be relaxed (e.g., the heart at diastole, and the left ventricle relaxed and filling with blood). Subsequently, at location 112, the particular portion of the heart wall has been activated and contraction of the myocardium at that location has commenced. This use of strain to determine mechanical activation activity for particular locations or regions of the heart wall can be more accurately indicate mechanical activation activity since such strain measurements can differentiate between the passive motion of the heart and the active motion of the heart (which is the true motion due to myocardial contraction). Using this technique, mechanical activation activity can be defined as the time when the strain reaches its minimum or some percentage of its minimum, the latter being represented by the location 112 in FIG. 14. This provides another option for a type of information (i.e., mechanical activation activity determined using strain) that may be displayed in images such as those shown in FIGS. 9-12 using, for example, overlaid colors and color patterns. That is, mechanical activation data determined using strain may be represented to a physician by superimposing colors on images or other anatomical representations of the heart.

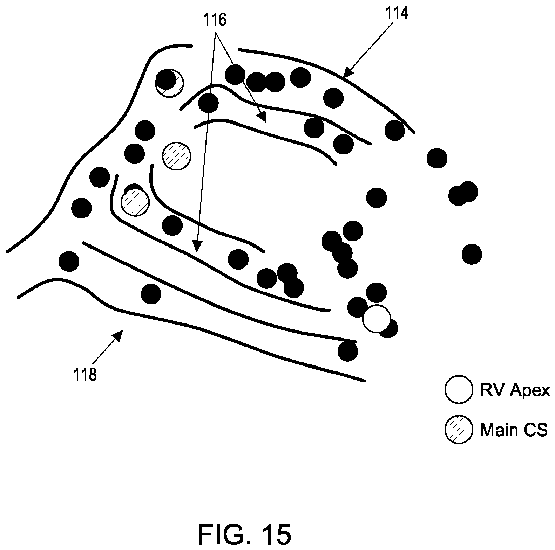

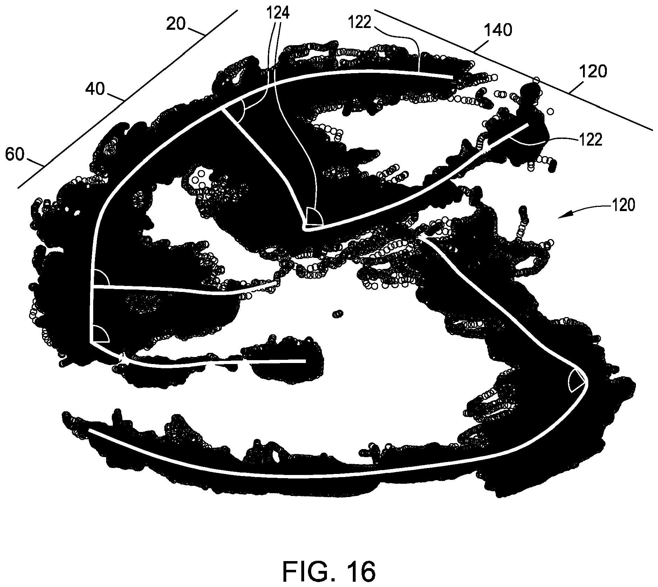

As already discussed, as a MediGuide-enabled tool (or some other trackable tool) is maneuvered around the coronary sinus and its branches, the 3D location of the MDG sensor is tracked. In various embodiments, including at least some of those discussed above (e.g., when calculating or determining mechanical activation activity using strain as represented in, for example, FIG. 11), it can prove beneficial to know which motion measurements were taken in a single vein (e.g., in a single CS branch). For example, if you want to calculate linear strain along a single CS branch, it would be necessary to be able to determine which of your data points were taken in that particular CS branch. Likewise, if you want to calculate strain across triangular regions of myocardium, it would be desirable to be able to select data points taken in different CS branches. Techniques for determining which MDG sensor data was tracked in which particular CS branch is further discussed below with reference to FIGS. 15 and 16.

FIG. 15 schematically depicts the difficulty that one may encounter when trying to determine which particular CS branch data was collected from using, for example, a MediGuide-enabled tool. In this figure, an anterior location 114, lateral locations 116, and an inferior location 118 for all identified; and each of the squiggly lines represents where veins may be located. Since the heart is beating and, thus, moving around during collection of the data at each of the motion points (represented by the black circles in FIG. 15), it can be extremely difficult to tell from the raw motion point data the vein (e.g., the particular CS branch) in which the location data was collected

The MediGuide system collects data the entire time that the MediGuide sensor is moving around in, for example, the coronary sinus vein tree. FIG. 16 depicts a sample resulting point cloud, showing many points, each selected from the same time during a plurality of cardiac cycles. In particular, each individual piece of motion data is represented in this figure by a small circle. Portions of the point cloud depicted in FIG. 16 appear to be completely black because of the density of the data (i.e., there are many overlapping circles) shown in this sample point cloud. For this technique, a cardiac cycle may be divided into bins using ECG data. Next, a particular bin is selected - either arbitrarily or, for example, the bin with the most number of points, or the bin encompassing diastole (when the heart is in its most stable state). Then, only the points in the selected bin are plotted. FIG. 16 is such a plot since, as above, it only contains points taken during the same time (or short window of time) during a plurality of cardiac cycles. An algorithm is applied to the data (e.g., one of the known skeletonization algorithms, including the distance transform, the Voronoi-skeleton, and thinning) to convert the point cloud into lines representing veins. The resulting lines representing veins are then analyzed to find turns in the lines and to measure angles 124 between lines.

Continuing to refer to FIG. 16, the angles 124 between lines 122 representing veins are analyzed. If the angles 124 are less than, for example, less than 100 degrees, then the technique may assume that the transition represented by these relatively "sharp turns" corresponds to connections between different veins. If, on the other hand the angles are not sharp (for example, or greater than 100 degrees), the technique may assume that these "soft turns" corresponds to movement down a single vein (e.g., down a single CS branch). Next, a number may be assigned to each branch of the skeleton that is separated by a relatively sharp turn from the other branches of the skeleton, thereby effectively assigning numbers to what has been determined to be separate veins. The MediGuide motion points are superimposed on the numbered skeleton and the 3D distance between each MediGuide motion point and the branches of the numbered skeleton is determined. Finally, each MediGuide motion point is assigned to the line that is closest to. In that manner, each MediGuide motion point is assigned to a particular vein.

In yet another technique for determining which motion points are in which particular veins, an assumption is made that motion points are collected in sequence, with the user going down one vein, then down another vein, then down another vein, etc. Next, the 3D distance between consecutive points can be calculated and analyzed. Whenever this 3D distance is above a predetermined threshold (for example, the mean distance between points plus the standard deviation of the distances, or based upon the size of the heart), an assumption is made that the user has moved to a different vein. In this matter, each of the motion points can be assigned to a particular vein.

Various embodiments are described herein to various apparatuses, systems, and/or methods. Numerous specific details are set forth to provide a thorough understanding of the overall structure, function, manufacture, and use of the embodiments as described in the specification and illustrated in the accompanying drawings. It will be understood by those skilled in the art, however, that the embodiments may be practiced without such specific details. In other instances, well-known operations, components, and elements have not been described in detail so as not to obscure the embodiments described in the specification. Those of ordinary skill in the art will understand that the embodiments described and illustrated herein are non-limiting examples, and thus it can be appreciated that the specific structural and functional details disclosed herein may be representative and do not necessarily limit the scope of the embodiments.

Although a number of embodiments have been described above with a certain degree of particularity, those skilled in the art could make numerous alterations to the disclosed embodiments without departing from the sprit or scope of this disclosure. For example, all joinder references (e.g., attached, coupled, connected, and the like) are to be construed broadly and may include intermediate members between a connection of elements and relative movement between elements. As such, joinder references do not necessarily infer that two elements are directly connected and in fixed relation to each other. It is intended that all matter contained in the above description or shown in the accompanying drawings shall be interpreted as illustrative only and not limiting. Changes in detail or structure may be made without departing from the spirit of the disclosure.

Reference throughout the specification to "various embodiments," "some embodiments," "one embodiment," or "an embodiment," or the like, means that a particular feature, structure, or characteristic described in connection with the embodiment is included in at least one embodiment. Thus, appearances of the phrases "in various embodiments," "in some embodiments," "in one embodiment," or "in an embodiment," or the like, in places throughout the specification are not necessarily all referring to the same embodiment. Furthermore, the particular features, structures, or characteristics may be combined in any suitable manner in one or more embodiments. Thus, the particular features, structures, or characteristics illustrated or described in connection with one embodiment may be combined, in whole or in part, with the features structures, or characteristics of one or more other embodiments without limitation given that such combination is not illogical or non-functional.

**Claims**

1. A system (10) capable of displaying mechanical activation patterns for a heart, the system comprising the following:

   a data input adapted to receive data from an electrophysiology apparatus (12);
   a processor electrically connected to the data input, the processor configured to execute the following steps:

   calculate mechanical activation parameters from the data;
   generate an anatomical representation of the heart from the data;
   divide the anatomical representation into segments (54); and
   generate a depiction that displays magnitudes of the mechanical activation parameter relative to the segments (54) such that performance of a plurality of segments (54) can be simultaneously evaluated; and

   an output adapted to transmit the depiction that displays magnitudes of the mechanical activation parameter relative to the segments to a display; and
   wherein the display is configured and arranged to facilitate clinician diagnosis based on the visualized magnitudes of the mechanical activation parameter relative to the segments (54), and the mechanical activation parameter comprises a time or displacement parameter.

2. The system (10) of claim 1, wherein the mechanical activation parameter is selected from the group consisting of heart wall displacement distance, heart wall displacement time, time when strain reaches a threshold, time when strain reaches its minimum, or time when strain reaches a specified percentage of its minimum.

3. The system (10) of claim 1 or 2, wherein the magnitude comprises a weighted average or a weighted standard deviation; the mechanical activation parameter is the time parameter which is indicative of the relative activation times of the segments (54); and the magnitudes of the mechanical activation parameter relative to the segments (54) are visualized as various shades, colors, or cross-hatching patterns.

4. The system (10) of any one of claims 1 to 3, wherein the depiction comprises a schematic illustration of the anatomical representation; the mechanical activation parameter is the displacement parameter which is indicative of the relative distance of each of the segments (54) between a nominal position and a maximum displacement location; and the magnitudes of the mechanical activation parameter relative to the segments (54) are visualized as various shades, colors, or cross-hatching patterns.

5. The system (10) of claim 4, wherein the illustration comprises a two-dimensional picture that shows aggregated mechanical activation data for each segment of the anatomical representation relative to a global view of the anatomical representation in a single view, or wherein the illustration comprises a bulls-eye plot or a data plot.

6. The system (10) of any one of claims 1 to 3, wherein the depiction comprises a rendering of the anatomical representation.

7. The system (10) of claim 6, wherein the rendering comprises a three-dimensional picture that shows aggregated mechanical activation data for each segment (54) of the heart relative to a global representation of the anatomical representation, wherein the rendering preferably comprises a three-dimensional image or a three dimensional model obtained via the electrophysiology apparatus (12).

8. The system (10) of any one of claims 1 to 7, wherein:

   the processor is configured to generate a three-dimensional model from the collected data; and
   the segments (54) of the heart are referenced on the three-dimensional model relative to anatomical markers, wherein the three-dimensional model preferably comprises an internal chamber of the heart, or
the processor is configured to generate a three-dimensional image from the collected data; and
the segments (54) of the heart are referenced on the three-dimensional image relative to anatomical markers,
wherein the three-dimensional image preferably comprises a coronary sinus having a plurality of branches (86A, 86B, 86C, 86D).

9.  A method for displaying mechanical activation patterns of a heart, the method comprising the following:

obtaining mechanical activation data points (52) at a processor from a data input communicatively coupled to the processor, the data points (52) correlated to locations on an anatomical representation;
dividing the anatomical representation into segments (54) representing different anatomical regions of the heart using the processor;
assigning each mechanical activation data point to at least one segment (54) using the processor;
analyzing mechanical activation data points (52) in each segment (54) using the processor;
displaying, on a display, a depiction representative of the magnitudes of the mechanical activation parameter relative to the segments (54), wherein each segment (54) is identified by an indicator representative of the analyzed mechanical activation data points (52), and the mechanical activation parameter comprises a time or displacement parameter.

10. The method of claim 9, wherein the indicator comprises a number, a color, or a column of data points (52).

11. The method of claim 9 or 10, wherein the depiction comprises a data plot and the indicator comprises columns of data points (52) for each segment (54), or
wherein the depiction comprises a bulls-eye plot and the indicator comprises color-coding of segments (54) of the bulls-eye plot.

12. The method of any one of claims 9 to 11, wherein:

the anatomical representation comprises a three-dimensional image;
the depiction comprises a rendering of the three-dimensional image bearing the indicator; and
the indicator comprises color-coding of different anatomical regions assigned to each segment (54), or
the anatomical representation comprises a three-dimensional model;
the depiction comprises a rendering of the three-dimensional model bearing the indicator; and
the indicator comprises color-coding of different anatomical regions assigned to each segment (54).

13. The method of any one of claims 9 to 12, further comprising identifying abnormal mechanical activation patterns in the segments (54).

14. The method of any one of claims 9 to 12, wherein dividing the anatomical representation into segments (54) comprises the following:

dividing a hull shape into a plurality of transverse slices; and
dividing each transverse slice into a plurality of equal longitudinal slices, or
identifying a plurality of branches (86A, 86B, 86C, 86D) in an organ; and
assigning each branch to a segment (54).

15. The method of claim 14, wherein dividing the anatomical representation into segments (54) further comprises dividing each branch into a plurality of segments (54).

**Patentansprüche**

1.  System (10), das geeignet ist zum Anzeigen von mechanischen Aktivierungsmustern für ein Herz, wobei das System Folgendes enthält:

einen Dateneingang, der zum Empfangen von Daten aus einer elektrophysiologischen Vorrichtung (12) angepasst ist;
einen Prozessor, der mit dem Dateneingang elektrische verbunden ist, wobei der Prozessor zum Ausführen der nachfolgenden Schritte ausgebildet ist:

Berechnen von mechanischen Aktivierungsparametern aus den Daten;
Erzeugen einer anatomischen Darstellung des Herzens aus den Daten;
Unterteilen der anatomischen Darstellung in Segmente (54); und
Erzeugen einer Abbildung, die Stärken der mechanischen Aktivierungsparameter relativ zu den Segmenten (54) derart zeigt, dass eine Leistung einer Vielzahl von Segmente (54) gleichzeitig bewertet werden kann; und

einen Ausgang, der zum Übertragen der Abbildung, die Stärken des mechanischen Aktivierungsparameters relativ zu den Segmenten zeigt, auf eine Anzeige angepasst ist; und
wobei die Anzeige ausgebildet ist und angeordnet ist zum Vereinfachen einer klinischen Diagnose, die auf den visualisierten Stärken des mechanischen Aktivierungsparameters relativ zu den Segmenten (54) basiert, und der mechanische Aktivierungsparameter einen Zeit- oder Verschiebeparameter aufweist.

2. System (10) nach Anspruch 1, wobei der mechanische Aktivierungsparameter aus der Gruppe ausgewählt wird, die aus einem Herzwand-Verschiebeabstand, einer Herzwand-Verschiebezeit, einer Zeit, wenn eine Belastung eine Schwellenwert erreicht, einer Zeit, wenn eine Belastung ihr Minimum erreicht, oder einer Zeit, wenn eine Belastung einen spezifizierten Prozentsatz ihres Minimums erreicht, besteht.

3. System (10) nach Anspruch 1 oder 2, wobei die Stärke ein gewichtetes Mittel oder eine gewichtete Standardabweichung aufweist; wobei der mechanische Aktivierungsparameter der Zeitparameter ist, der die relativen Aktivierungszeiten der Segmente (54) angibt; und die Stärken des mechanischen Aktivierungsparameters relativ zu den Segmenten (54) als verschiedene Schatten, Farben oder Kreuzschraffierungsmuster visualisiert werden.

4. System (10) nach einem der Ansprüche 1 bis 3, wobei die Abbildung eine schematische Illustration der anatomischen Darstellung enthält; der mechanische Aktivierungsparameter der Verschiebeparameter ist, der den relativen Abstand jedes der Segmente (54) zwischen einer Nominalposition und einer Maximalverschiebestelle angibt; und die Stärken des mechanischen Aktivierungsparameters relativ zu den Segmenten (54) als verschiedene Schatten, Farben oder Kreuzschraffierungsmuster visualisiert werden.

5. System (10) nach Anspruch 4, wobei die Illustration ein zweidimensionales Bild enthält, das angehäufte mechanische Aktivierungsdaten für jedes Segment der anatomischen Darstellung relativ zu einer Globalansicht der anatomischen Darstellung in einer einzigen Ansicht zeigt, oder wobei die Illustration einen Bulls-Eye-Plot oder einen Datenplot enthält.

6. System (10) nach einem der Ansprüche 1 bis 3, wobei die Abbildung ein Rendering der anatomischen Darstellung enthält.

7. System (10) nach Anspruch 6, wobei das Rendering ein dreidimensionales Bild enthält, das angehäufte mechanische Aktivierungsdaten für jedes Segment (54) des Herzens relativ zu einer globalen Darstellung der anatomischen Darstellung zeigt, wobei das Rendering vorzugsweise ein dreidimensionales Bild oder ein dreidimensionales Modell enthält, das über die elektrophysiologische Vorrichtung (12) erhalten wird.

8. System (10) nach einem der Ansprüche 1 bis 7, wobei
der Prozessor zum Erzeugen eines dreidimensionalen Modells aus den gesammelten Daten ausgebildet ist; und
die Segmente (54) des Herzens sich auf das dreidimensionale Modell relativ zu anatomischen Markern beziehen, wobei das dreidimensionale Modell vorzugsweise eine Innenkammer des Herzens enthält, oder
der Prozessor zum Erzeugen eines dreidimensionalen Bildes aus den gesammelten Daten ausgebildet ist; und
die Segmente (54) des Herzens sich auf das dreidimensionale Bild relativ zu anatomischen Markern beziehen, wobei das dreidimensionale Bild vorzugsweise einen Koronarsinus enthält, der eine Vielzahl von Verzweigungen (86A, 86B, 86C, 86D) enthält.

9. Verfahren zum Anzeigen von mechanischen Aktivierungsmustern eines Herzens, wobei das Verfahren Folgendes enthält:

Erhalten von mechanischen Aktivierungsdatenpunkten (52) an einem Prozessor aus einem Dateneingang, der mit dem Prozessor kommunikativ gekoppelt ist, wobei die Datenpunkte (52) Stellen auf einer anatomischen Darstellung zugeordnet sind;

Unterteilen der anatomischen Darstellung in Segmente (54), die verschiedene anatomische Regionen des Herzens wiedergeben, unter Verwendung des Prozessors;

Zuordnen jedes mechanischen Aktivierungsdatenpunkts zu wenigstens einem Segment (54) unter Verwendung des Prozessors;

Analysieren der mechanischen Aktivierungsdatenpunkte (52) in jedem Segment (54) unter Verwendung des Prozessors;

Anzeigen auf einer Anzeige einer Abbildung, die die Stärken des mechanischen Aktivierungsparameters relativ zu den Segmenten (54) wiedergibt, wobei jedes Segment (54) durch einen Indikator identifiziert wird, der die analysierten mechanischen Aktivierungsdatenpunkte (52) wiedergibt, und der mechanische Aktivierungsparameter einen Zeit- oder Verschiebeparameter aufweist.

**10.** Verfahren nach Anspruch 9, wobei die Abbildung eine Anzahl, eine Farbe oder eine Kolonne von Datenpunkten (52) enthält.

**11.** Verfahren nach Anspruch 9 oder 10, wobei der Indikator einen Datenplot enthält und der Indikator Kolonnen von Datenpunkten (52) für jedes Segment (54) enthält, oder

wobei die Abbildung einen Bulls-Eye-Plot enthält und der Indikator eine Farbkodierung von Segmenten (54) des Bully-Eye-Plots enthält.

**12.** Verfahren nach einem der Ansprüche 9 bis 11, wobei:

die anatomische Darstellung ein dreidimensionales Bild enthält;
die Abbildung ein Rendering des dreidimensionalen Bildes enthält, das den Indikator trägt; und
der Indikator eine Farbkodierung verschiedener anatomischer Regionen aufweist, die jedem Segment (54) zugeordnet ist, oder
die anatomische Darstellung ein dreidimensionales Modell enthält;
die Abbildung ein Rendering des dreidimensionalen Modells enthält, das den Indikator trägt; und
der Indikator eine Farbkodierung verschiedener anatomischer Regionen enthält, die jedem Segment (54) zugeordnet ist.

**13.** Verfahren nach einem der Ansprüche 9 bis 12, ferner enthaltend das Identifizieren von abnormalen mechanischen Aktivierungsmustern in den Segmenten (54).

**14.** Verfahren nach einem der Ansprüche 9 bis 12, wobei das Unterteilen der anatomischen Darstellung in Segmente (54) Folgendes enthält:

Unterteilen einer Rumpfform in eine Vielzahl von Querscheiben; und
Unterteilen jeder Querscheibe in eine Vielzahl von gleichen Längsscheiben, oder
Identifizieren einer Vielzahl von Verzweigungen (86A, 86B, 86C, 86D) in einem Organ; und
Zuordnen jeder Verzweigung zu einem Segment (54).

**15.** Verfahren nach Anspruch 14, wobei das Unterteilen der anatomischen Darstellung in Segmente (54) ferner das Unterteilen jeder Verzweigung in eine Vielzahl von Segmente (54) enthält.

## Revendications

**1.** Système (10) capable d'afficher des schémas d'activation mécanique pour un coeur, le système comprenant les éléments suivants :

une entrée de données adaptée pour recevoir des données d'un appareil d'électrophysiologie (12) ;
un processeur connecté électriquement à l'entrée de données, le processeur étant configuré pour exécuter les étapes consistant à :

calculer les paramètres d'activation mécanique à partir des données ;
générer une représentation anatomique du coeur à partir des données ;
diviser la représentation anatomique en segments (54) ; et

générer une représentation qui affiche les valeurs du paramètre d'activation mécanique par rapport aux segments (54) de telle sorte que les performances d'une pluralité de segments (54) peuvent être évaluée simultanément ; et

une sortie adaptée pour transmettre la représentation qui affiche sur un dispositif d'affichage les valeurs du paramètre d'activation mécanique par rapport aux segments ; et

dans lequel le dispositif d'affichage est configuré et agencé pour faciliter le diagnostic du clinicien sur la base des valeurs visualisées du paramètre d'activation mécanique par rapport aux segments (54), et le paramètre d'activation mécanique comprend un paramètre de temps ou de déplacement.

2. Système (10) selon la revendication 1, dans lequel le paramètre d'activation mécanique est choisi dans le groupe constitué par la distance de déplacement de la paroi cardiaque, le temps de déplacement de la paroi cardiaque, le moment où la tension atteint un seuil, le moment où la tension atteint son minimum ou le moment où la tension atteint un pourcentage spécifique de son minimum.

3. Système (10) selon la revendication 1 ou 2, dans lequel la valeur comprend une moyenne pondérée ou un écart-type pondéré ; le paramètre d'activation mécanique est le paramètre temporel qui est indicatif des temps d'activation relatifs des segments (54) ; et les valeurs du paramètre d'activation mécanique par rapport aux segments (54) sont visualisées sous différentes nuances, couleurs ou schémas d'hachures croisées.

4. Système (10) selon l'une quelconque des revendications 1 à 3, dans lequel la représentation comprend une illustration schématique de la représentation anatomique ; le paramètre d'activation mécanique est le paramètre de déplacement qui est indicatif de la distance relative de chacun des segments (54) entre une position nominale et un emplacement de déplacement maximum ; et les valeurs du paramètre d'activation mécanique par rapport aux segments (54) sont visualisées sous différentes nuances, couleurs ou schémas d'hachures croisées.

5. Système (10) selon la revendication 4, dans lequel l'illustration comprend une image bidimensionnelle qui montre des données d'activation mécanique agrégées pour chaque segment de la représentation anatomique par rapport à une vue globale de la représentation anatomique dans une vue unique, ou dans lequel l'illustration comprend une représentation graphique en couronne concentriques ou une représentation graphique de données.

6. Système (10) selon l'une quelconque des revendications 1 à 3, dans lequel la représentation comprend un rendu de la représentation anatomique.

7. Système (10) selon la revendication 6, dans lequel le rendu comprend une image tridimensionnelle qui montre des données d'activation mécanique agrégées pour chaque segment (54) du coeur par rapport à une représentation globale de la représentation anatomique, dans lequel le rendu comprend de préférence une image tridimensionnelle ou un modèle tridimensionnel obtenu via l'appareil d'électrophysiologie (12).

8. Système (10) selon l'une quelconque des revendications 1 à 7, dans lequel :

le processeur est configuré pour générer un modèle tridimensionnel à partir des données recueillies ; et les segments (54) du coeur sont référencés sur le modèle tridimensionnel par rapport aux marqueurs anatomiques, dans lequel le modèle tridimensionnel comprend de préférence une chambre interne du coeur, ou le processeur est configuré pour générer une image tridimensionnelle à partir des données recueillies ; et les segments (54) du coeur sont référencés sur l'image tridimensionnelle par rapport à des marqueurs anatomiques, dans lequel l'image tridimensionnelle comprend de préférence un sinus coronaire ayant une pluralité de branches (86A, 86B, 86C, 86D).

9. Procédé d'affichage de schémas d'activation mécanique d'un coeur, le procédé comprenant les étapes consistant à :

obtenir des points de données d'activation mécanique (52) au niveau d'un processeur à partir d'une entrée de données couplée de manière communicative au processeur, les points de données (52) étant corrélés à des emplacements sur une représentation anatomique ;

diviser la représentation anatomique en segments (54) représentant de différentes régions anatomiques du coeur à l'aide du processeur;

assigner chaque point de données d'activation mécanique à au moins un segment (54) à l'aide du processeur ;

analyser des points de données d'activation mécanique (52) dans chaque segment (54) à l'aide du processeur;

afficher, sur un dispositif d'affichage, une représentation représentative des valeurs du paramètre d'activation mécanique par rapport aux segments (54), dans lequel chaque segment (54) est identifié par un indicateur représentatif des points de données (52) d'activation mécanique analysés, et le paramètre d'activation mécanique comprend un paramètre de temps ou de déplacement.

10. Procédé selon la revendication 9, dans lequel l'indicateur comprend un nombre, une couleur ou une colonne de points de données (52).

11. Procédé selon la revendication 9 ou 10, dans lequel la représentation comprend un graphique de données et l'indicateur comprend des colonnes de points de données (52) pour chaque segment (54), ou
dans lequel la représentation comprend une représentation graphique en couronne concentriques et l'indicateur comprend un codage couleur des segments (54) de la représentation graphique en couronne concentriques.

12. Procédé selon l'une quelconque des revendications 9 à 11, dans lequel :

la représentation anatomique comprend une image tridimensionnelle ;
la représentation comprend un rendu de l'image tridimensionnelle portant l'indicateur ; et
l'indicateur comprend un code couleur de différentes régions anatomiques assignées à chaque segment (54), ou
la représentation anatomique comprend un modèle tridimensionnel ;
la représentation comprend un rendu du modèle tridimensionnel portant l'indicateur ; et
l'indicateur comprend un code couleur de différentes régions anatomiques assignées à chaque segment (54).

13. Procédé selon l'une quelconque des revendications 9 à 12, comprenant en outre l'identification de schémas d'activation mécanique anormaux dans les segments (54).

14. Procédé selon l'une quelconque des revendications 9 à 12, dans lequel la division de la représentation anatomique en segments (54) comprend les étapes consistant à :

diviser une forme de coque en une pluralité de tranches transversales ; et
diviser chaque tranche transversale en une pluralité de tranches longitudinales égales, ou
identifier une pluralité de branches (86A, 86B, 86C, 86D) dans un organe ; et
assigner chaque branche à un segment (54).

15. Procédé selon la revendication 14, dans lequel la division de la représentation anatomique en segments (54) comprend en outre la division de chaque branche en une pluralité de segments (54).

FIG. 1

EP 3 113 680 B1

FIG.2

FIG.3

FIG.4

FIG.5

PATIENT 15 MAX DISP

FIG. 6

FIG. 7

FIG. 8

EP 3 113 680 B1

Left | Right | 25 ▼

80

88
Early | Late

86C
86D
86B
84
82
86A

( x )
cine #1
LAO 0  CR 0

( )
cine #2
LAO 0  CR 0

( )
cine #3
LAO 44 CR 0

( x )
cine #4
LAO 0  CR 0

( ✓ )
cine #5
LAO 0  CR 0

( )
cine #6
LAO 44  CR 0

90

90

cm 25

86E

[Cine #5] [0018] Zoom: 1.0 SID: 99 LAO:3 CR:0

Turn
Magnet
OFF

FIG. 9

FIG. 10

FIG. 11

FIG. 12

Left | Right | 25 ▾

cine #1
LAO 0 CR 0

cine #2
LAO 0 CR 0

cine #3
LAO 44 CR 0

cine #4
LAO 0 CR 0

cine #5
LAO 0 CR 0

cine #6
LAO 44 CR 0

Early                    Late

88

104

102

108

106

110

cm 25

[Cine #5] [0008] Zoom: 0.9 SID: 99 LAO:3 CR:0

Turn
Magnet
OFF

80

90

30

EP 3 113 680 B1

FIG. 13

FIG. 14

FIG. 15

FIG. 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7263397 B, Hauck
- US 2009254140 A1
- US 2014005563 A1
- US 6233476 B, Strommer
- US 20080183071 A, Strommer
- US 7386339 B, Strommer
- US 8195292 B, Rosenberg

### Non-patent literature cited in the description

- Three dimensional mapping of mechanical activation patterns, contractile dyssynchrony and dyscoordination by two-dimensional strain echocardiography: Rationale and design of a novel software box. **DE BOECK BART WL et al.** CARIOVASCULAR ULTRASOUND. BIOMED CENTRAL, 30 May 2008, vol. 6, 1476-7120